(19) Europäisches Patentamt
European Patent Office
Office européen des brevets

(11) Numéro de publication: **0 034 521**
**B1**

(12) # FASCICULE DE BREVET EUROPEEN

(45) Date de publication du fascicule du brevet: **23.03.83**

(51) Int. Cl.³: **C 07 D 307/82,** A 61 K 31/495

(21) Numéro de dépôt: **81400173.1**

(22) Date de dépôt: **04.02.81**

(54) **Médicament analgésique contenant de la 1-(3'benzofuryl)-4-benzylpipérazine et son procédé de préparation.**

(30) Priorité: **14.02.80 FR 8003284**

(43) Date de publication de la demande:
**26.08.81 Bulletin 81/34**

(45) Mention de la délivrance du brevet:
**23.03.83 Bulletin 83/12**

(84) Etats contractants désignés:
**AT BE CH DE GB IT LI NL SE**

(56) Documents cités:
**FR-A-2 421 900**
**JOURNAL OF ORGANIC CHEMISTRY, vol. 38, no. 9, 4 mai 1973 WASHINGTON (US) C. K. TSENG et al.: »Synthesis of aminobenzofurans and aminonaphto (1,2-b) furans«, pages 1746, 1747**

(73) Titulaire: **Buzas, André, 25 route de Versailles, F-91570 Bievres (FR)**
Titulaire: **Melon, Jean-Marie, 158, rue de Courcelles, F-75017 Paris (FR)**
Titulaire: **LABORATOIRES SAUBA S.A. Société anonyme française, 260 rue de Rosny, F-93104 Montreuil (FR)**
Titulaire: **UNIVERSITE D'ORLEANS (Etablissement français d'enseignement supérieur à caractère scientifique, et culturel) Château de la Source, F-45046 Orleans Cedex (FR)**

(72) Inventeur: **Buzas, André, 25 route de Versailles, F-91570 Bievres (FR)**
Inventeur: **Melon, Jean-Marie, 158 rue de Courcelles, F-75017 Paris (FR)**
Inventeur: **Lavielle, Gilbert, 38 rue du 11-novembre, F-45000 Orleans (FR)**
Inventeur: **Champagnac, André, 13, rue de la Chaussée, F-60510 Bresles (FR)**

(74) Mandataire: **Bouju, André, 38 Avenue de la Grande Armée, F-75017 Paris (FR)**

EP 0 034 521 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

# Médicament analgésique contenant de la 1-(3'-benzofuryl)-4-benzylpipérazine et son procédé de préparation

La présente invention concerne un médicament analgésique renfermant en tant que substance active un dérivé de la benzylpipérazine et son procédé de préparation.

On connaît de nombreux médicaments analgésiques.

La demande de brevet français FR-A-2 421 900 décrit des pipérazino-3-indoles doués de propriétés analgésiques et répondant à la formule générale suivante:

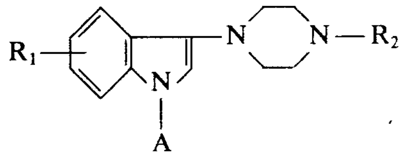

dans laquelle:

A est un atome d'hydrogène, un radical acide carboxylique ou un groupement alkyle, alkylamino alkyle, benzylique ou phényle, substitué ou non,

$R_1$ est un atome d'hydrogène ou de chlore ou un groupement alkyle inférieur, méthoxy ou hydroxy,

$R_2$ est un groupement alkyle, benzylique ou phényle ou un groupement cyclique ou hétérocyclique, substitué ou non.

Parmi ces composés, le plus actif est le 1-acétyl-3 benzylpipérazino indole. Ce composé possède des propriétés analgésiques remarquables et comparables à celles de la morphine.

Suivant la présente invention, les déposants ont découvert un nouveau médicament renfermant en tant que substance active un dérivé de la benzylpipérazine et présentant des propriétés analgésiques qui sont supérieures à celles du composé connu précité et à celles de la morphine et sans présenter les inconvénients connus de cette dernière.

Suivant l'invention, ce médicament analgésique est caractérisé en ce qu'il renferme en tant que substance active de la 1-(3'-benzofuryl)-4-benzylpipérazine.

Ce composé présente la formule développée suivante:

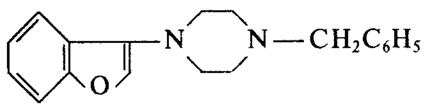

Ce composé se présente à l'état pur sous la forme de cristaux dont le point de fusion est de 120° C.

La formule brute de composé conforme à l'invention s'écrit: $C_{19}H_{18}N_2O$. L'analyse élémentaire de ce composé a fourni les résultats suivants:

| | | | |
|---|---|---|---|
| % théorique: | C 78,05 | H 6,90 | N 9,58 |
| % trouvé: | C 78,28 | H 6,87 | N 9,59 |

En résonance magnétique nucléaire (RMN), dans du $CDCl_3$, on obtient les résultats suivants:

7,6–7,0 (10 H, m, 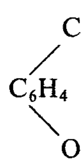 $C_6H_5$, O—CH=C)

3,5 (2 H, s, $CH_2$—$C_6H_5$)
3,2–2,9 (4 H, m) et
2,8–2,5 (4 H, m) (4 N—$CH_2$)

En chromatographie, en couche mince on obtient les résultats suivants:

Rf = 0,50 (20% d'éther dans du chlorure de méthylène)
Rf = 0,75 (10% de méthanol dans du chlorure de méthylène)

Pour préparer le 1-(3'-benzofuryl)-4-benzylpipérazine, on procède conformément à l'invention, de la façon suivante: on fait réagir sous reflux de la 3-coumaranone sur de la N-benzylpipérazine, en présence d'acide acétique et dans un solvant aromatique.

De préférence, on utilise un léger excès de N-benzylpipérazine par rapport à la quantité

stoechiométrique théoriquement nécessaire.

On donne ci-après à titre d'exemple numérique, non limitatif, un mode opératoire détaillé de préparation du composé conforme à l'invention.

On mélange 8 g (0,06 mole) de 3-coumaranone, 12,6 g (0,072 mole) de N-benzylpipérazine et 1,2 ml d'acide acétique dans 180 ml de toluène sec.

Le mélange ci-dessus est mis à reflux sous agitation, dans un réacteur équipé d'un séparateur Dean-Stark, pendant 8 heures. On entraîne ainsi environ 1 ml d'eau. La plus grande partie du solvant (toluène) est évaporée sous vide. La résidu obtenu est lavé plusieurs fois avec de l'eau, par trituration, puis dissous dans 100 ml d'alcool éthylique bouillant.

Après refroidissement, on essore le précipité, on le lave à nouveau avec de l'alcool éthylique et on recristallise le produit dans 200 ml d'isopropanol en présence de noir animal.

Après filtration, on lave avec un petit volume d'isopropanol et on sèche le produit solide, sous vide, à 40°C. On obtient ainsi 8,5 g de produit conforme à l'invention, soit avec un rendement de 48% par rapport au produit de départ.

On va maintenant exposer les propriétés pharmaceutiques du composé conforme à l'invention. Ces propriétés sont comparées ci-après avec le 1-acétyl-3-benzylpipérazine indole qui est décrit dans la demande de brevet français FR-A-2 421 900.

Toxicité aigüe chez la Souris (DL50)

La toxicité aigüe est évaluée d'après la mortalité observée pendant 48 heures sur des lots de 4 souris ayant reçu par voie orale 100, 200, 400, 800 et 1600 mg/kg du composé étudié. On obtient ainsi les résultats suivants:

Composé connu:
DL50 = 3000 micro-moles par Kg (1000 mg/kg)
Composé conforme à l'invention:
DL50 = 5500 micro-moles par Kg (1600 mg/kg)

La toxicité aigüe du composé conforme à l'invention est par conséquent inférieure à celle du dérivé connu.

Activité analgésique

Test de Siegmund à la phénylbenzoquinone chez la souris

Ce test a été effectué selon la méthode décrite par Siegmund, Cadmus et Lu (»A method for evaluating both non-narcotic and narcotic analgesics«, Proc. Soc. Exp. Biol. Med., 1957, 95, 729—731).

Une heure après l'administration du produit étudié, par voie orale, à des lots de 12 souris par dose, la phénylbenzoquinone, en solution à raison de 0,02% dans de l'eau contenant 5% d'alcool éthylique, est injectée par voie intra-péritonéale, à raison de 0,25 ml par souris de 20 g.

On dénombre les torsions présentées par chaque animal entre la cinquième et la dixième minute qui suivent l'injection de la phénylbenzoquinone.

La DE50 (dose diminuant de 50% le nombre de torsions par rapport aux animaux témoins) est calculée à partir de la relation dose/effet. On obtient ainsi les résultats suivants:

Composé connu:
DE50 = 55 micro-moles par Kg (18 mg/kg)
Dérivé conforme à l'invention:
DE50 = 22 micro-moles par Kg (6,5 mg/kg).

Les résultats de ce test mettent donc en évidence une plus grande efficacité pour le produit conforme à l'invention.

Test de la plaque chauffante chez la Souris (Test d'EDDY)

Ce test a été effectué selon la méthode décrite par N. B. EDDY (J. Pharmacol; 1932, 45, 339—359).

Une heure après l'administration du produit étudié, par voie orale, à des lots de 12 souris par dose, on relève le temps que mettent les souris, placées sur une plaque chauffée à 56,5°C à se lécher les pattes antérieures.

La DE30 (dose augmentant de 30% le temps de léchage par rapport aux témoins) est calculée à partir de la relation dose/effet.

On obtient ainsi les résultats suivants:

Dérivé connu:
DE30 = 40 micro-moles par Kg (13 mg/kg)
Dérivé conforme à l'invention:
70 micro-moles par Kg (20 mg/kg)

### Antagonisme par la Naloxone

Cet antagonisme est recherché sur le test de Siegmund à la phénylbenzoquinone chez la souris, tel que décrit précédemment. La Naloxone est administrée à une dose fixe par voie sous-cutanée, en même temps que le produit étudié. On obtient ainsi les résultats suivants:

pour le composé connu (en présence de 16 micro moles par Kg de Naloxone):
DE50 = 45 micro-moles par Kg (15 mg/kg)
pour le dérivé conforme à l'invention (en présence de 8 micro moles par Kg de Naloxone):
DE50 = 46 micro-moles par Kg (13,5 mg/kg)

### Intéractions in vitro

### Action sur des organes isolés

Ces intéractions sont recherchées sur des organes isolés, maintenus en survie dans un liquide nutritif. Les contractions de ces organes sont enregistrées sur un kymographe par un mécanisme isotonique.

Un agoniste est ajouté au bain de liquide nutritif, seul ou après ajout d'un antagoniste, à des concentrations croissantes permettant de réaliser des courbes dose-réponse cumulatives.

Le paramètre d'affinité d'un agoniste (pDx), la nature compétitive ou non-compétitive d'un antagonisme et le paramètre d'affinité d'un antagoniste compétitif (pAx) sont estimés à partir de l'étude qualitative et quantitative des familles de courbes dose-réponse.

### Antagonisme vis-à-vis de l'histamine

Dans ce test, l'organe sur lequel a été étudiée cette action, est l'Iléon de cobaye le liquide nutritif utilisé étant de la Tyrode, l'agoniste est l'histamine et l'antagoniste est le produit étudié.

On a ainsi obtenu les résultats suivants:

pour le produit connu: antagoniste non-compétitif.

pour le produit conforme à l'invention: antagoniste compétitif, le paramètre d'affinité ($pA_2$) ayant été trouvé égal à 5,99.

### Antagonisme vis-à-vis de la sérotonine

L'organe étudié est le Fundus de rat, le liquide nutritif utilisé est le liquide de Kreps, l'agoniste est la sérotonine et l'antagoniste est le produit étudié. On a ainsi obtenu les résultats suivants:

pour le composé connu: antagoniste compétitif, ($pA_2 = 5{,}34$) pour le dérivé conforme à l'invention: antagoniste non-compétitif.

### Agonisme sur le Fundus de rat

L'organe étudié est le Fundus de rat, le liquide nutritif, le liquide de Kreps, et l'agoniste le produit étudié.

On a obtenu les résultats suivants:

pour le dérivé connu: paramètre d'affinité ($pD_2$) = 5,4
pour le dérivé conforme à l'invention: paramètre d'affinité ($pD_2$) = 7,0.

Antagonisme par l'harmine

L'organe étudié dans ce test est le Fundus de rat, le liquide nutritif, le liquide de Kreps, l'agoniste correspond au produit étudié et l'antagoniste est l'harmine.

On a ainsi trouvé les résultats suivants: vis-à-vis du produit connu, l'harmine présente un antagonisme non-compétitif et vis-à-vis du produit conforme à l'invention, l'harmine présente un antagonisme compétitif, le paramètre d'affinité ($pA_2$) ayant été trouvé égal à 6,85.

Fixation sur les récepteurs cérébraux aux morphiniques

L'affinité des produits étudiés est recherchée par leur capacité de déplacement de l'étorphine tritiée dans un homogénat de cerveau de rat (à 1 mg/ml de protéine), pendant 30 mn 35° C. Dans ce test, on détermine le CI50 qui correspond à la concentration de produits capables d'inhiber 50% de la liaison spécifique du ligande radioactif. On a ainsi trouvé les résultats suivants:

Produit connu: 103 000 nmol/l
Produit conforme à l'invention: supérieur à 300 000 nmol/l

A titre de comparaison, la concentration CI50 de la morphine est de 179 nmol/l.

Les résultats des essais pharmacologiques précités montrent que le produit conforme à l'invention est un composé présentant en particulier par son action dans le test d'EDDY, des propriétés analgésiques centrales telles que définies dans l'article de D. R. LAURENCE et A. L. BACHARACH, (Evaluation of Drug Activities: Pharmocometrics, Academic Press London and New York, 1964), mais aucune liaison avec les récepteurs aux morphiniques ne peut être mis en évidence. Les propriétés générales du composé conforme à l'invention sont comparables à celles du produit connu utilisé dans les tests précités, avec toutefois le fait que, les propriétés analgésiques du produit conforme à l'invention, sont sensiblement supérieures. Cependant, d'une manière surprenante et imprévisible, les deux composés diffèrent fondamentalement quant à leur action au niveau moléculaire. Ce fait est bien mis en évidence par leurs activités radicalement opposées dans les tests d'antagonisme par la Naloxone et d'intéractions in vitro, sur organes isolés. En effet, selon les résultats des tests d'antagonisme par la Naloxone, le produit conforme à l'invention est antagonisé par la Naloxone, tandis que le composé connu ne l'est pas. De même, selon le test d'intéractions, in vitro sur organes isolés, le composé conforme à l'invention est un antagoniste compétitif de l'histamine, non-caompétitif de la sérotonine et il est antagonisé compétitivement par l'harmine. Par contre, le produit connu est un antagoniste compétitif de la sérotonine, non-compétitif de l'histamine et il n'est pas antagonisé compétitivement par l'harmine.

Par conséquent, le 1-(3'-benzofuryl)-4-benzylpipérazine et le 1-acétyl-3-benzylpipérazino indole connu sont deux composés analgésiques dont les modes d'action sont tout à fait différents.

Par ailleurs, l'intérêt du composé conforme à l'invention par rapport à la morphine, est que pour le composé conforme à l'invention, il n'existe pas de risque de dépendance comme dans le cas de la morphine.

Ainsi, grâce à ces remarquables propriétés analgésiques, le médicament conforme à l'invention est utilisable pour le traitement de toutes les affections nécessitant l'emploi d'un analgésique aussi puissant que la morphine.

Le médicament conforme à l'invention peut être administré par voie orale, par exemple sous forme de comprimé ou de gellule, par voie rectale, sous forme de suppositoire ou par voie intra-musculaire, sous forme de soluté injectable conditionné dans des ampoules.

Lors de l'utilisation par voie orale, le médicament conforme à l'invention, est administré à une dose comprise 10 à 20 mg de substance active par prise, la posologie quotidienne étant de préférence comprise entre 30 et 100 mg de produit.

Lors de l'administration par voie rectale, on utilisera de préférence des suppositoires dosés à 25 mg de substance active à raison de 3 suppositoires par jour.

Lors de l'administration par voie intra-musculaire, les ampoules de soluté injectable seront de préférence dosées à raison de 20 mg de substance active, les injections étant effectuées à raison de une à deux par jour.

On donne ci-après quelques exemples de formes pharmaceutiques du médicament conforme à l'invention:

— Comprimé:
  composé conforme à l'invention . . . 10 mg
  polyvinylpyrrolidone, amidon de blé, talc, stéarate . . . Q. S. P. pour un comprimé de 150 mg.
— Gellule:
  composé conforme à l'invention . . . 10 mg
  polyvinylpyrrolidone, amidon de blé, aérosil . . . Q. S. P. pour un comprimé de 150 mg.

— Suppositoire:
composé conforme à l'invention . . . 25 mg
eau pour disperser à 60° dans des glycérides semisynthétiques Q. S. P. pour un suppositoire de 2 g environ.

— Soluté injectable:
composé conforme à l'invention . . . 20 mg à l'état lyophilisé dans une ampoule.
acide ascorbique . . . 70 mg
eau . . . 1 ml, ces deux composés étant conditionnés dans une ampoule distincte destinée à être mélangée au moment de l'emploi avec l'ampoule contenant le composé conforme à l'invention.

**Revendications pour les Etats Contractants: BE, Ge/Ü, DE, GB, IT, NL, SE**

1. Médicament analgésique renfermant en tant que substance active un dérivé de la benzylpipérazine, caractérisé en ce que cette substance active est constituée par la 1-(3'-benzofuryl)-4-benzylpipérazine.
2. Médicament conforme à la revendication 1, caractérisé en ce qu'il est dosé en vue de son usage médicinal à un poids de substance active compris entre 10 et 20 mg.
3. Procédé pour la préparation de la 1-(3'-benzofuryl)-4-benzylpipérazine, caractérisé en ce qu'on fait réagir sous reflux de la 3-coumaranone sur de la N-benzylpipérazine, en présence d'acide acétique et dans un solvant aromatique.
4. Procédé conforme à la revendication 3, caractérisé en ce qu'on utilise un léger excès de N-benzylpipérazine par rapport à la quantité stoechiométrique théoriquement nécessaire.

**Revendications pour l'Etat Constractant: AT**

1. Procédé pour la préparation de la 1-(3'-benzofuryl)-4-benzylpipérazine, caractérisé en ce qu'on fait réagir sous reflux de la 3-coumaranone sur de la N-benzylpipérazine, en présence d'acide acétique et dans un solvant aromatique.
2. Procédé conforme à la revendication 1, caractérisé en ce qu'on utilise un léger excès de N-benzylpipérazine par rapport à la quantité stoechiométrique théoriquement nécessaire.

**Patentansprüche für die Vertragsstaaten: BE, Ge/Ü, DE, GB, IT, NL, SE**

1. Schmerzstillendes Arzneimittel, das als Wirkstoff ein Benzylpiperazin-Derivat enthält, dadurch gekennzeichnet, daß dieser Wirkstoff durch 1-(3'-Benzofuryl)-4-benzylpiperazin gebildet ist.
2. Arzneimittel nach Anspruch 1, dadurch gekennzeichnet, daß für seine medizinische Anwendung der Anteil des Wirkstoffs zwischen 10 und 20 mg dosiert ist.
3. Verfahren zur Herstellung von 1-(3'-Benzofuryl)-4-benzylpiperazin, dadurch gekennzeichnet, daß 3-Kumaranon rückfließend auf das N-Benzylpiperazin in Gegenwart von Essigsäure und einem aromatischen Lösungsmittel einwirkt.
4. Verfahren nach Anspruch 3, dadurch gekennzeichnet, daß ein gegenüber dem theoretisch erforderlichen stöchiometrischen Wert ein geringer Überschuß von N-Benzylpiperazin verwendet wird.

**Patentansprüche für den Vertragsstaat: AT**

1. Verfahren zur Herstellung des neuen 1-(3'-Benzofuryl)-4-benzyl-piperazins, dadurch gekennzeichnet, daß 3-Kumaranon rückfließend auf das N-Benzylpiperazin in Gegenwart von Essigsäure und einem aromatischen Lösungsmittel einwirkt.
2. Verfahren nach Anspruch 1, dadurch gekennzeichnet, daß ein gegenüber dem theoretisch erforderlichen stöchiometrischen Wert ein geringer Überschuß von N-Benzylpiperazin verwendet wird.

**Claims for the contracting states: BE, Ge/Ü, DE, GB, IT, NL, SE**

1. An analgesic drug containing a derivative of benzylpiperazine as active substance, wherein said active substance is constituted by 1-(3'-benzofuryl)-4-benzylpiperazine.
2. A drug according to claim 1, wherein said drug is dosed for medicinal use at a weight of active substance within the range of 10 to 20 mg.
3. A method for the preparation of 1-(3'-benzofuryl)-4-benzylpiperazine, wherein a reaction is

conducted with reflux between 3-coumaranone and N-benzylpiperazine in the presence of acetic acid and in an aromatic solvent.

4. A method according to claim 3, wherein the quantity of N-benzylpiperazine employed is slightly in excess with respect to the stoichiometric quantity which is theoretically necessary.

**Claims for the contracting state: AT**

1. A method for the preparation of 1-(3′-benzofuryl)-4-benzylpiperazine, wherein a reaction is conducted with reflux between 3-coumaranone and N-benzylpiperazine in the presence of acetic acid and in an aromatic solvent.

2. A method according to claim 1, wherein the quantity of N-benzylpiperazine employed is slightly in excess with respect to the stoichiometric quantity which is theoretically necessary.